(19) Europäisches Patentamt
European Patent Office
Office européen des brevets

(11) **EP 3 086 861 B1**

(12) **EUROPEAN PATENT SPECIFICATION**

(45) Date of publication and mention
of the grant of the patent:
**20.02.2019 Bulletin 2019/08**

(21) Application number: **13819170.5**

(22) Date of filing: **23.12.2013**

(51) Int Cl.:
*A61Q 11/00* (2006.01)     *A61K 8/81* (2006.01)
*A61K 8/22* (2006.01)

(86) International application number:
**PCT/US2013/077388**

(87) International publication number:
**WO 2015/099643 (02.07.2015 Gazette 2015/26)**

(54) **WHITENING ORAL CARE COMPOSITIONS**

AUFHELLENDE MUNDPFLEGEZUSAMMENSETZUNGEN

COMPOSITIONS DE SOINS BUCCAUX POUR BLANCHIMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.11.2016 Bulletin 2016/44**

(73) Proprietor: **Colgate-Palmolive Company**
**New York, NY 10022 (US)**

(72) Inventors:
• **MALONEY, Venda Porter**
  **Piscataway, New Jersey 08854 (US)**
• **CHOPRA, Suman K.**
  **Monroe, New Jersey 08831 (US)**
• **ONTUMI, Dennis**
  **Easton, Pennsylvania 18045 (US)**
• **MANDADI, Prakasarao**
  **Flemington, New Jersey 08822 (US)**

(74) Representative: **Wibbelmann, Jobst et al**
**Wuesthoff & Wuesthoff**
**Patentanwälte PartG mbB**
**Schweigerstrasse 2**
**81541 München (DE)**

(56) References cited:
**WO-A1-2012/102750     WO-A2-2013/095369**
**US-A1- 2013 165 530     US-A1- 2013 287 710**

**Description**

**BACKGROUND**

Many individuals are dissatisfied with their current tooth color. Thus, there is a desire for whiter teeth and one means to achieve whiter teeth is the use of tooth whitening products.

The teeth can become discolored by foods, drinks and tobacco use. Dental stains can be classified as either extrinsic, which occur on the outer surface of' teeth, or intrinsic, which occur below the surface of enamel. Most abrasive containing toothpaste remove extrinsic stains. Hydrogen peroxide can bleach both extrinsic and intrinsic stains and so provides faster and superior whitening efficacy. The peroxide can bleach the teeth, remove stains, and kill cariogenic bacteria. However, peroxide compounds are highly reactive, and consequently difficult to formulate. Moreover, hydrogen peroxide can spontaneously decompose to form oxygen gas ($O_2$) and water, so that on storage, the dentifrice containers may bloat, burst or leak, and the remaining formulation will not have enough peroxide remaining to clean and whiten teeth effectively. Some dentifrices initially comprise very high levels of peroxide, which decomposes over time, so that the exact amount of peroxide delivered on application is variable and largely depends on how long and under what conditions the dentifrice has been stored.

WO 2013/095369 A2 discloses a non-aqueous dental whitening composition comprising a peroxide component in an amount of 0.1 % to 5 % of the total weight.

US 2003/165530 A1 is directed to a foamable alcoholic composition comprising inter alia (example 4) polyvinylpyrrolidone and $H_2O_2$.

WO 2012/102750 A1 discloses a dentifrice composition comprising a polyethylene glycol co-polymer, glycerin, and cross-linked PVP/$H_2O_2$.

**BRIEF SUMMARY**

The present invention concerns a dentifrice composition comprising a whitening complex comprising crosslinked polyvinylpyrrolidone complexed with hydrogen peroxide, comprising the following ingredients by weight - wherein the total of the ingredients by weight may not exceed 100% :

| | |
|---|---|
| Glycerin | 10-60 wt% |
| Propylene glycol | 0.01-60 wt% |
| Ethylene oxide, propylene oxide block copolymer, avg. MW > 5kDa | 0.01-15% |
| Crosslinked polyvinylpyrrolidone complexed with 15-25% hydrogen peroxide | 0.275-1.1 wt% |
| Calcium pyrophosphate | 0.01-45 wt% |
| Tetrasodium pyrophosphate (TSPP) | 0.01-5 wt% |

Further embodiments are set forth in the attached claims.

**DETAILED DESCRIPTION**

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The term "wt%" is an abbreviation for weight percent. The amounts given are based on the active weight of the material.

In some embodiments the compositions of the invention further comprise an abrasive. In some embodiments, the abrasive is a calcium abrasive.

By exposure to aqueous environments, as in the oral cavity, the PVP-$H_2O_2$ dissociates into individual species (PVP polymer and $H_2O_2$). The PVP- $H_2O_2$ complex is generally comprised of about 80% by weight polyvinyl pyrrolidone and 20% by weight $H_2O_2$. Single phase whitening dentifrice compounds comprising PVP- $H_2O_2$ complexes are described, e.g., in WO/2007/037961, and its parent US Pub. No. US 2007-0071695 A1.

| Humectants, e.g. Glycerin | 10-60%, or 20-50%, e.g., about 35% |
|---|---|
| Propylene glycol | 0.01-60%, or 5-25% e.g., about 15% |
| Thickeners, e.g., Fumed silica | 0-3%, e.g., about 1.75% |
| Polymers, e.g., Ethylene oxide, propylene oxide block co-polymer, avg. MW >5kDa | 0.01-15%, or 5-12%, e.g., about 7.5% |
| Polyethylene glycol 600 | 0-15% or 1-15%, e.g., about 6% |
| Polyvinylpyrrolidone | 0-10% or 1-10%, e.g., 5.75% |
| Optionally, additional linear and/or crosslinked polyvinylpyrrolidone | 0.25-10 wt% |
| Crosslinked polyvinylpyrrolidone complexed with 15-25% hydrogen peroxide | 0.275-1.1%, or, 0.4125-0.825%, e.g., about 0.55% |
| Abrasive, Calcium pyrophosphate | 0.01-45%, or about 5-30%, e.g., about 20% |
| Fluoride, Sodium monofluorophosphate | 0-2% or about 0.1-1.5%, e.g. about 1.1% |
| Surfactant, e.g., SLS | 0-3%, or 0.1-3%, e.g., about 2% |
| Tartar control agent, TSPP | 0.01-5%, or 0.1-4% e.g., about 2% |
| Antioxidant, e.g. BHT | 0.01-5%, e.g., about 0.03% |
| Flavorings | 0.1 - 5, e.g., 1.4% |
| Phosphoric acid | 0.01-3%, e.g., 0.2% |
| Water | <3% |

The compositions of the invention a "low water" content, meaning that a total concentration of water, including any free water and all water contained in any ingredients, is less than about 5%, preferably less than 3%, preferably less than 2% water.

The average particle size of the abrasives is generally about 0.1 to about 30 microns, for example about 1 to about 20 or about 5 to about 15 microns.

The oral care composition can optionally include at least one orally acceptable source of fluoride ions. Any known or to be developed in the art may be used. Suitable sources of fluoride ions include fluoride, monofluorophosphate and fluorosilicate salts. One or more fluoride ion-releasing compound is optionally present in an amount providing a total of about 100 to about 20,000 ppm, about 200 to about 5,000 ppm, or about 500 to about 2,500 ppm, fluoride ions.

The compositions of the invention may also comprise various dentifrice ingredients to adjust the rheology and feel of the composition such as humectants, surface active agents, thickening or gelling agents, etc.

The compositions of the present invention may comprise a surface active agent (surfactant). Suitable, surfactants include without limitation water-soluble salts of $C_{8-20}$ alkyl sulfates, sulfonated monoglycerides of $C_{8-20}$ fatty acids, sarcosinates, taurates, sodium lauryl sulfate, sodium cocoyl monoglyceride sulfonate, sodium lauryl sarcosinate, sodium lauryl isoethionate, sodium laureth carboxylate and sodium dodecyl benzenesulfonate, and cocoamidopropyl betaine.

It is preferred that the carrier(s) provide a dentifrice with a viscosity of about 10,000 CPS to about 700,000 CPS, preferably about 30,000 CPS to about 300,000 CPS.

As recognized by one of skill in the art, the oral compositions of the present invention optionally include other materials, such as for example, anti-caries agents, desensitizing agents, viscosity modifiers, diluents, surface active agents, such as surfactants, emulsifiers, and foam modulators, pH modifying agents, abrasives, in addition to those listed above, humectants, mouth feel agents, sweetening agents, flavor agents, colorants, preservatives, and combinations thereof. It is understood that while general attributes of each of the above categories of materials may differ, there may be some common attributes and any given material may serve multiple purposes within two or more of such categories of materials. Preferably; the carrier is selected for compatibility with other ingredients of the composition.

Flavorants, sweeteners, colorants, foam modulators, mouth-feel agents and others additively may be included if desired, in the composition.

The compositions of the present invention optionally comprise one or more further active material(s), which is operable for the prevention or treatment of a condition or disorder of hard or soft tissue of the oral cavity, the prevention or treatment of a physiological disorder or condition, or to provide a cosmetic benefit.

The compositions may include a stannous ion or a stannous ion source. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of about 0.01% to about 10%, for example about 0.1% to about 7% or about 1% to about 5%.

The compositions of the present invention optionally comprise an antimicrobial (e.g., antibacterial) agent. A further illustrative list of useful antibacterial agents is provided in such as those listed in U.S. Pat. No. 5,776,435 to Gaffar et al. One or more antimicrobial agents are optionally present in an antimicrobial effective total amount, typically about 0.05% to about 10%, for example about 0.1% to about 3%.

The compositions of the present invention optionally comprise an antioxidant. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

The compositions of the present invention optionally comprise a sialagogue or saliva-stimulating agent, an antiplaque agent, an anti-inflammatory agent, and/or a desensitizing agent.

While ingredients are sometimes identified herein by category, e.g., humectant, antioxidant, thickener, etc., this identification is for convenience and clarity, but is not intended to be limiting. All of the ingredients in the compositions may have functions in addition to their primary function, and may contribute to the overall properties of the composition, including its stability, efficacy, consistency, mouthfeel, taste, odor and so forth.

pH modifying agents among those useful herein include acidifying agents to lower pH, basifying agents to raise pH, and buffering agents to control pH within a desired range. For example, one or more compounds selected from acidifying, basifying and buffering agents can be included to provide a pH of 2 to 10, or in various embodiments from 2 to 8, from 3 to 9, from 4 to 8, from 5 to 7, from 6 to 10, and from 7 to 9. Any orally acceptable pH modifying agent can be used, including without limitation carboxylic, phosphoric and sulfonic acids, acid salts (e.g., monosodium citrate, disodium citrate, monosodium malate, etc.), alkali metal hydroxides such as sodium hydroxide, carbonates such as sodium carbonate, bicarbonates, sesquicarbonates, borates, silicates, phosphates (e.g., monosodium phosphate, trisodium phosphate, pyrophosphate salts, etc.), imidazole and mixtures thereof. One or more pH modifying agents are optionally present in a total amount effective to maintain the composition in an orally acceptable pH range.

The product form of the compositions of the invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

Methods are provided to whiten a tooth surface in a human or animal subject comprising contacting a composition of the invention, as described above, and with the tooth surface. In one embodiment the composition remains stable when stored for at least 1 week, at least 2 weeks, at least 1 month, at least 3 months, at least 6 months, or at least 1 year prior to contacting with the tooth surface. In one embodiment the composition is stored at room temperature. As used herein "animal subject" includes higher order non-human mammals such as canines, felines, and horses. The oral care composition is contacted with an oral surface of the mammalian subject to thereby whiten teeth in a highly efficacious manner, without any negative interaction between the whitening agent, the blue to blue-violet dye, and other ingredients.

In various embodiments, it is preferred that the oral care composition is applied and contacted with the oral surface. The dentifrice, prepared in accordance with the present invention is preferably applied regularly to an oral surface, preferably on a daily basis, at least one time daily for multiple days, but alternately every second or third day. Preferably the oral composition is applied to the oral surfaces from 1 to 3 times daily, for at least 2 weeks up to 8 weeks, from four months to three years, or more up to lifetime.

The compositions of the invention can be packaged into containers or dispensers known in the art, via means conventional in the art. In some embodiments the compositions are packaged into tubes, metal, plastic or laminated, with either screw top or flip top caps.

In some embodiments, the diameter of the top of the tube in which the a composition of the present invention is packaged, expands less than 0.1 cm, after 1 week of aging at 60°C. While in other embodiments, the diameter of the top of the tube in which a composition of the present invention is packaged, does not expand to a measurable extent.

In some embodiments, the compositions of the present invention do not exhibit an unacceptable level of phase separation, e.g., after 30 minutes at 2050 rpm in a LumiSizer 110 analytical centrifuge.

The invention is illustrated in the following non-limiting examples.

4

## EXAMPLES

### Example 1

Comparison of a low peroxide toothpaste with a commercial whitening toothpaste

The product formulas included a low peroxide toothpaste composition shown in Table 1 and a commercial whitening silica toothpaste shown in Table 2:

**Table 1**

|  | Range (%) | Actual (%) |
|---|---|---|
| Glycerin | 0.01 - 60 | 36.41 |
| L1220 | 0.01 - 10 | 7.5 |
| PG | 0.01 - 60 | 15 |
| PEG 600 | 0 - 15 | 6.31 |
| PVP.HP | 0.05 - 0.55 | 0.55 |
| Calcium pyrophosphate | 0.01 - 40 | 20 |
| Fluoride | 0 - 1.1 | 1.1 |
| XPVP | 0 - 10 | 5.75 |
| Fumed Silica | 0.01 - 10 | 1.75 |
| SLS | 0-4 | 2 |
| Flavor | 0.01 - 4.0 | 1.4 |
| Water | 0 - 3 | 0 |
| TSPP | 0.01 - 5 | 2 |
| BHT | 0.01-0.1 | 0.03 |
| Phosphoric Acid | 0.01-3 | 0.2 |
| Total | 100 | 100 |

**Table 2**

|  | Actual (%) |
|---|---|
| Xanthan Gum | 0.2 |
| Sodium CMC | 0.5 |
| Gylcerin | 19.94 |
| Sorbitol | 16.00 |
| PEG 600 | 3 |
| Sachharin | 0.3 |
| Sodium Fluoride | 0.32 |
| TKPP | 2.44 |
| STPP | 3.0 |
| Sodium Hydroxide -25% | 0.54 |
| Water | 19.16 |
| Silica VP5 | 22 |

(continued)

|  | Actual (%) |
|---|---|
| Thickener (Silica) | 4.5 |
| SLS slurry | 5.17 |
| Betaine | 1.25 |
| Flavor | 1.44 |
| Blue Poly 50 | 0.2 |
| Pigment blue | 0.02 |
| Total | 100 |

*In-vitro Testing Procedure*

In-vitro whitening efficacy of the two products was tested by brushing experiments consisting of the following test procedure.

• Prophy artificially stained bovine enamel to achieve similar initial lightness values

• Mount four bovine teeth into a tray

• Prepare 1:1 (w/w) slurry of dentifrice to DI water and add 25 grams to brushing tray

• Brush the stained bovine teeth for 2 minutes at 120 strokes/min

• Rinse teeth with DI water then evaluate for L*a*b values with spectrophotometer

• Repeat for 28 cycles (equivalent to 1 month product use)

*Spectrophotometer Metrics*

CIELAB is most commonly used color scale
L* value: Lightness of a color
L*=0 black and L*=100 white

$$\Delta L^* = L^*\text{brushed} - L^*\text{initial}$$

a* value: +a* = magenta and -a* = green
b* value: +b* = yellow and -b* = blue
W value:

- Incorporates the L*, a* and b* values to describe how close the measured color is to true white

- $W^* = (a^{*2} + b^{*2} + (L^*-100)^2)^{1/2}$

The data below in Table 1 is reporting change in W* value after treatment (ΔW*), Larger *negative* ΔW* value = whiter

**Table 3**

|  | Week 1 | Week 2 | Week 3 | Week 4 |
|---|---|---|---|---|
| Low Peroxide (0.1 %) Toothpaste | 7.8 ± 3.2 | 9.1 ± 3.5 | 9.5 ± 3.4 | 9.8 ± 3.3 |
| Commercial whitening Silica Toothpaste with stain prevention ingredients | 4.7 ± 1.9 | 5.7± 2.0 | 7.3 ± 2.4 | -7.6 ± 2.6 |

6

Table 3 shows ΔW low peroxide toothpaste vs commercial whitening silica toothpaste with stain prevention ingredients.

The data below in Table 4 is reporting change in ΔL*

- Larger ΔL* value = whiter
- ΔL* = L*brushed - Linitial

**Table 4**

|  | Week 1 | Week 2 | Week 3 | Week 4 |
|---|---|---|---|---|
| Low Peroxide (0.1%) Toothpaste | 7.0 ± 2.9 | 8.2 ± 3.0 | 8.5 ± 2.9 | 8.9 + 2.9 |
| Commercial whitening Silica Toothpaste with stain prevention ingredients | 4.4 ± 1.7 | 5.4 ± 1.8 | 7.2 ± 2.6 | -7.7 ± 2.6 |

Table 4 shows ΔL* low peroxide toothpaste vs commercial whitening silica toothpaste with stain prevention ingredients.

The data belowi Table 5 is reporting change in Δb*

- B* value: +b* = yellow and -b* = blue

**Table 5**

|  | Week 1 | Week 2 | Week 3 | Week 4 |
|---|---|---|---|---|
| Low Peroxide (0.1%) Toothpaste | -3.1 ± 1.6 | -3.7 ± 1.9 | [-3.9 ± 2.0 | [-3.9 ± 1.8 |
| Commercial whitening Silica Toothpaste with stain preventention ingredients | -1.5 ± 1.0 | -1.8 ± 1.2 | -1.8 ± 1.6 | -1.7 ± 1.9 |

Table 5 shows Δb* Low Peroxide toothpaste vs Commercial whitening Silica Toothpaste with stain prevention ingredients.

The data below in Table 6 is reporting change in Δa

- a* value: +a* = magenta and -a* = green

**Table 6**

|  | Week 1 | Week 2 | Week 3 | Week 4 |
|---|---|---|---|---|
| Low Peroxide (0.1%) Toothpaste | -1.5 ± 0.5 | 1.6+ 0.6 | -1.9 ± 0.8 | -1.71± 0.7 |
| Commercial whitening Silica Toothpaste with stain preventention ingredients | -0.7± 0.6 | -0.8± 0.7 | -1.0 ± 0.7 | -1.0± 0.8 |

Table 6 shows Δa* Low Peroxide toothpaste vs Commercial whitening Silica Toothpaste with stain preventention ingredients

The ΔW*, ΔL*, Δb* and Δa* values in Tables 3, 4, 5 and 6 is the summary of testing results for teeth treated with low peroxide toothpaste and teeth treated with a commercial whitening silica containing toothpaste with stain prevention ingredients.

## Example 2

0.1% HP peroxide formula delivers greater whitening efficacy than a commercial whitening silica containing toothpaste with stain prevention ingredients

*Consumer Test Results*

The objective of the consumer test was to screen new whitening toothpaste prototypes vs a commercial whitening silica toothpaste with stain prevention ingredients. In the monadic study consumers evaluated product and provided opinions on whitening performance after product use. In this study the consumers were asked to report when they noticed tooth whitening. The results of these study is shown below in Table 7. The low peroxide toothpaste with 0.1% HP was found to provide whitening by significantly more panelists at week 1 and week 2 of the study.

**Table 7**

|  | Low Peroxide (0.1%) [Toothpaste | Commercial whitening Silica Toothpaste with stain preventention ingredient |
|---|---|---|
| **Week 1** | 43% | 17% |
| **Week 2** | 2% | 47% |

Table 7 shows percentage of respondents that saw whitening in the low peroxide vs a Commercial whitening Silica Toothpaste with stain prevention ingredient

Table 8 below illustrates how consumers ranked the products for shininess. The change is reported as change in perceived tooth shine from pretrial of product. There is a significant difference between the change in shine rankings for the 0.1% HP toothpaste vs. the Commercial Whitening SilicaToothpaste with stain prevention ingredients.

**Table 8**

|  | Change after 2 weeks | Change after 4 weeks |
|---|---|---|
| Low Peroxide (0.1%) Toothpaste | 1.06 | 1.59 |
| Commercial whitening silica toothpaste with stain prevention ingredient | 0.65 | 1.02 |

Table 8 shows low peroxide formula achieves much higher increase in shineness than the commercial whitening silica toothpaste with stain prevention ingredient. 0.1% HP peroxide formula delivers greater whitening and shine efficacy than a commercial whitening silica toothpaste with stain prevention ingredient.

## Claims

1. A dentifrice composition comprising a whitening complex comprising crosslinked polyvinylpyrrolidone complexed with hydrogen peroxide, comprising the following ingredients by weight - wherein the total of the ingredients by weight may not exceed 100% :

| Glycerin | 10-60 wt% |
|---|---|
| Propylene glycol | 0.01-60 wt% |
| Ethylene oxide, propylene oxide block copolymer, avg. MW > 5kDa | 0.01-15% |
| Crosslinked polyvinylpyrrolidone complexed with 15-25% hydrogen peroxide | 0.275-1.1 wt% |
| Calcium pyrophosphate | 0.01-45 wt% |
| Tetrasodium pyrophosphate (TSPP) | 0.01-5 wt% |

EP 3 086 861 B1

2. The composition of claim 1 wherein the ethylene oxide, propylene oxide block co-polymer comprises (ethylene oxide)x-(propylene oxide)y wherein x is an integer of 80-150 and y is an integer 30-80.

3. The composition of claims 1 or 2 wherein the ethylene oxide, propylene oxide co-polymer has an average molecular weight of greater than 5000 Da and is free of an ethylene oxide, propylene oxide block co-polymer of average molecular weight less than 5000 Da.

4. The composition of any of the preceding claims additionally comprising polyethylene glycol of average molecular weight 400 to 800 Da.

5. The composition of any of the preceding claims which contains less than 3% water by weight.

6. The composition of any of the preceding claims comprising additional linear and/or crosslinked polyvinylpyrrolidone.

7. The composition of claim 1 comprising:

| | |
|---|---|
| Glycerin | 20-50 wt% |
| Propylene glycol | 5-25 wt% |
| Ethylene oxide, propylene oxide block copolymer, avg. MW > 5kDa | 5-12 wt% |
| Additional linear and/or crosslinked polyvinylpyrrolidone | 0.25-10 wt% |
| Crosslinked polyvinylpyrrolidone complexed with 15-25% hydrogen peroxide | 0.275-1.1 wt % |
| Calcium pyrophosphate | 5-30 wt% |
| TSPP | 0.1-4 wt% |

8. The composition of claim 1 comprising the following ingredients:

| | |
|---|---|
| Ethylene oxide, propylene oxide block co-polymer | 7.5 wt % |
| calcium pyrophosphate | 20 wt% |
| polyvinylpyrolidone complexed with hydrogen peroxide | 0.55 wt% |

**Patentansprüche**

1. Zahnputzmittelzusammensetzung, umfassend einen Aufhellungskomplex, umfassend quervernetztes Polyvinylpyrrolidon, komplexiert mit Wasserstoffperoxid, umfassend die folgenden Bestandteile in Gewichtsanteilen, wobei die Gesamtheit der Bestandteile als Gewichtsanteil 100 % nicht übersteigen:

| | |
|---|---|
| Glycerin | 10-60 Gew.-% |
| Propylenglycol | 0,01-60 Gew.-% |
| Ethylenoxid, Propylenoxid-Block-Copolymer, durchschnittliches MW > 5 kDa | 0,01-15 Gew.-% |
| Quervernetztes Polyvinylpyrrolidon, komplexiert mit 15-25 % Wasserstoffperoxid | 0,275-1,1 Gew.-% |
| Calciumpyrophosphat | 0,01-45 Gew.-% |
| Tetranatriumpyrophosphat (TSPP) | 0,01-5 Gew.-% |

2. Zusammensetzung nach Anspruch 1, worin das Ethylenoxid, Propylenoxid-Block-Copolymer (Ethylenoxid)x-(Propylenoxid)y umfasst, worin x eine Ganzzahl von 80-15 und y eine Ganzzahl von 30-80 ist.

3. Zusammensetzung nach Ansprüchen 1 oder 2, worin das Ethylenoxid, Propylenoxid-Copolymer ein mittleres Molekulargewicht von größer als 5 000 Da hat und frei von Ethylenoxid, Propylenoxid-Block-Copolymer mit einem mittleren Molekulargewicht geringer als 5 000 Da ist.

4. Zusammensetzung nach irgendeinem der vorhergehenden Ansprüche, zusätzlich umfassend Polyethylenglycol mit einem mittleren Molekulargewicht von 400 bis 800 Da.

5. Zusammensetzung nach irgendeinem der vorhergehenden Ansprüche, die weniger als 3 Gew.-% Wasser enthält.

6. Zusammensetzung nach irgendeinem der vorhergehenden Ansprüche, zusätzlich umfassend lineares und/oder quervernetztes Polyvinylpyrrolidon.

7. Zusammensetzung nach Anspruch 1:

| Glycerin | 20-50 Gew.-% |
|---|---|
| Propylenglycol | 5-25 Gew.-% |
| Ethylenoxid, Propylenoxid-Block-Copolymer, durchschnittliches MW > 5 kDa | 5-12 Gew.-% |
| Zusätzliches lineares und/oder quervernetztes Polyvinylpyrrolidon | 0,25-10 Gew.-% |
| Quervernetztes Polyvinylpyrrolidon, komplexiert mit 15-25 % Wasserstoffperoxid | 0,275-1,1 Gew.-% |
| Calciumpyrophosphat | 5-30 Gew.-% |
| TSPP | 0,1-4 Gew.-% |

8. Zusammensetzung nach Anspruch 1, umfassend die folgenden Bestandteile:

| | |
|---|---|
| Ethylenoxid, Propylenoxid-Block-Copolymer | 7,5 Gew.-% |
| Calciumpyrophosphat | 20 Gew.-% |
| Polyvinylpyrrolidon, komplexiert mit Wasserstoffperoxid | 0,55 Gew.-%. |

**Revendications**

1. Composition de dentifrice comprenant un complexe de blanchiment comprenant de la polyvinylpyrrolidone réticulée complexée avec du peroxyde d'hydrogène, comprenant les ingrédients suivants en poids - dans laquelle le total en poids des ingrédients ne peut pas excéder 100 % :

| Glycérine | 10 à 60 % en poids |
|---|---|
| Propylène glycol | 0,01 à 60 % en poids |
| Copolymère séquencé d'oxyde d'éthylène et d'oxyde de propylène, MW moy. > 5 kDa | 0,01 à 15 % |
| Polyvinylpyrrolidone réticulée, complexée avec 15 à 25 % de peroxyde d'hydrogène | 0,275 à 1,1 % en poids |
| Pyrophosphate de calcium | 0,01 à 45 % en poids |
| Pyrophosphate tétrasodique (TSPP) | 0,01 à 5 % en poids |

2. Composition selon la revendication 1, dans laquelle le copolymère séquencé d'oxyde d'éthylène et d'oxyde de propylène comprend (oxyde d'éthylène)x-(oxyde de propylène)y, dans lequel x est un nombre entier de 80 à 150 et y est un nombre entier de 30 à 80.

3. Composition selon la revendication 1 ou 2, dans laquelle le copolymère d'oxyde d'éthylène et d'oxyde de propylène a un poids moléculaire moyen supérieur à 5 000 Da et est dépourvu de copolymère séquencé d'oxyde d'éthylène et d'oxyde de propylène d'un poids moléculaire moyen inférieur à 5 000 Da.

**4.** Composition selon l'une quelconque des revendications précédentes, comprenant en outre du polyéthylène glycol d'un poids moléculaire moyen de 400 à 800 Da.

**5.** Composition selon l'une quelconque des revendications précédentes, qui contient moins de 3 % en poids d'eau.

**6.** Composition selon l'une quelconque des revendications précédentes, comprenant une polyvinylpyrrolidone linéaire et/ou réticulée supplémentaire.

**7.** Composition selon la revendication 1, comprenant :

| | |
|---|---|
| Glycérine | 20 à 50 % en poids |
| Propylène glycol | 5 à 25 % en poids |
| Copolymère séquencé d'oxyde d'éthylène et d'oxyde de propylène, MW moy. > 5 kDa | 5 à 12 % en poids |
| Polyvinylpyrrolidone linéaire et/ou réticulée supplémentaire | 0,25 à 10 % en poids |
| Polyvinylpyrrolidone réticulée, complexée avec 15 à 25 % de peroxyde d'hydrogène | 0,275 à 1,1 % en poids |
| Pyrophosphate de calcium | 5 à 30 % en poids |
| TSPP | 0,1 à 4 % en poids |

**8.** Composition selon la revendication 1, comprenant les ingrédients suivants : Copolymère séquencé d'oxyde d'éthylène et

| | |
|---|---|
| d'oxyde de propylène | 7,5 % en poids |
| Pyrophosphate de calcium | 20 % en poids |
| Polyvinylpyrolidone complexée avec du peroxyde d'hydrogène | 0,55 % en poids |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013095369 A2
- US 2003165530 A1
- WO 2012102750 A1
- WO 2007037961 A
- US 20070071695 A1
- US 5776435 A, Gaffar